# EUROPEAN PATENT APPLICATION

(11) **EP 2 835 054 A1**
(43) Date of publication of application: **11.02.2015**
(21) Application number: 13179259.0
(22) Date of filing: 05.08.2013
(51) Int. Cl.: A01N 43/90, A61L 2/18, C02F 1/50, A01P 1/00

(54) **Porphyrinoid compounds, method and apparatus for water photodisinfection.**

(71) Applicant: Nimartech Limited, London W1T 6LQ (GB)
(72) Inventor: Guidolin, Laura, I-36023 LONGARE (VI) (IT); Coppellotti, Olimpia, I-35132 PADOVA (IT)
(74) Representative: Spadaro, Marco

(57) **Abstract**

The present invention relates to disinfection of water polluted by microbial pathogens through the use of photosensitizers. The photosensitizer is a porphyrinoid which is part of an adduct comprising a resin, a spacer and said porphyrinoid. An apparatus wherein said adduct is excited by light sources and water is disinfected while flowing inside the apparatus is also provided. A system for the disinfection of water comprising said apparatus and further elements is also within the scope of the invention.

## Description

### Field of the invention

The present invention relates to disinfection of water polluted by potentially dangerous populations of microbial pathogens. More in particular, the invention relates to the disinfection of water by means of porphyrinoids immobilized on an inert resin and electronically excited by exposition to illumination with visible light.

### Background of the invention

Water performs a multiplicity of roles, since it can act as a reaction and transport medium, a temperature regulator and an important support for life processes as we know them. As human population and the number of human activities increase, it becomes more and more difficult to provide supplies of high-quality pure water from surface and ground water stocks. Actually, a growing number of countries around the world, especially developing countries but also several Mediterranean countries, face increasingly difficult situations concerning the availability of irrigation, industrial, and drinking water. Many water sources are heavily polluted by hazardous chemicals, which are known to be harmful to humans and a variety of animal and vegetal ecosystems, as well as by several kinds of pathogenic microorganisms, such as bacteria, fungi, molds, viruses, and parasitic protozoa.

Therefore, efficacious and environmentally safe techniques yielding an efficient water purification and disinfection are strongly needed.

Currently, the most commonly adopted methods of water disinfection are based on physical methods (e.g., filtration, heating, or irradiation with ultraviolet (UV) light or X-rays, even though reliance is mainly placed on chemical treatments with oxidizing species, such as chlorine, chlorine dioxide, or ozone (Tomas C.R., 1990, Wastewater engineering and treatment: problems and solutions. In: Chemical Engineering Techniques in Biotechnology, ed. M.A. Winkler, 23-94. Amsterdam: Elsevier). However, such methods often exhibit a number of negative aspects, in particular the formation of endoperoxides or trihalomethanes as by-products of the reaction between the chemical disinfectant and organic matter, or the selection of multidrug resistant microbial strains upon repeated treatment of waters characterized by a large microbial flora. Furthermore, in the case of aquaculture waters, where the large density of fish in the farming structures coupled with the high concentration of food residues, organic debris and defecation products often result in the formation of a large population of microbial pathogens, the water purification measures are based on the introduction into the fish farming tanks of chemicals, such as malachite green, formaldehyde or bronopol, that are potentially quite dangerous for the fish, the consumers and the environment (Magaraggia et al., 2006, Treatment of microbiologically polluted aquaculture waters by a novel photochemical technique of potentially low environmental impact. J. Environ. Monit. 8: 923-931).

The evolvement of resistance by microbial cells represents a particularly challenging problem as a consequence of the truly large variety of mechanisms, which have been found to be developed by pathogenic agents in order to increase their defensive strategies against external insults: these include a thickening of their outer wall, encoding of new proteins that prevent the penetration of drugs, onset of mutants deficient in those porin channels allowing the influx of externally added chemicals, etc. (Smith, T.L. et al., 1999, Emergence of vancomycin resistance in Staphylococcus aureus: glycopeptide-intermediate Staphylococcus aureus working group. New Engl. J. Med. 340: 493-501). The transfer of such resistance to human pathogens or other components of the various ecosystems represents a potentially serious threat to public health (Cabello, F.C., 2006, Heavy use of prophylactic antibiotics in aquaculture: a growing problem for human and animal health and for the environment. Environ. Microbiol. 8: 1137-1144).

Therefore, there is an urgent need for the development of convenient and low cost methods for combating water contamination, especially that caused by biological agents which can promote the onset of dangerous epidemic diseases.

The use of photochemical techniques for the treatment of chemically or microbiologically contaminated waters has been operative for many years with so far contrasting results. The effectiveness of UV light is often limited by the reduced penetration power (<1 cm) of wavelengths shorter than 350 nm into natural water; moreover, the direct absorption of UV-C and UV-B wavelengths by nucleic acids is a main cause of the onset of mutagenic processes leading to the selection of possibly dangerous new kinds of microbial strains (Mitchell, D.L. and D. Kerentz, 1993, The induction and repair of DNA photodamage in the environment. In: Environmental UV Photobiology, eds. A.R. Young et al, 345-378. New York: Plenum Press). To address these problems, novel photochemical pathways for water purification have been devised, including the introduction of photoassisted reactions via semiconductors (e.g., TiO₂ or ZnO), which can be electronically excited by light wavelengths in the UV-A spectral range giving raise to the formation of strongly oxidizing and highly cytotoxic species, *in primis* the OH radical and hydrogen peroxide. However, their usefulness is presently confined to small-scale water treatment, mainly owing to the reduced water volumes that can be illuminated in a homogeneous way by using light wavelengths in the UV-A spectral range, as well as to the relatively high costs associated with the operation of this technology. Furthermore, the oxidizing intermediates normally involved in such photoprocesses display an appreciable reactivity against most nucleotides, hence there is again a high probability of promoting mutagenic effects.

New perspectives have been opened by the treatment of waters with a high degree of microbial contamination by photodynamic processes, namely processes induced by the combined action of visible light wavelengths, oxygen and a photosensitising dye (Magaraggia M. and G. Jori, 2012, Photodynamic approaches to water disinfection. In: CRC Handbook of Organic Photochemistry and Photobiology, eds. A. Griesbeck et al., 1557-1570. Boca Raton: CRC Press). A variety of studies (see, for an exhaustive review, Jori G. et al., 2006, Photodynamic therapy in the treatment of microbial infections: basic principles and prospective applications. Lasers Surg. Med. 38: 468-481) consistently point out that the antimicrobial properties of photodynamic sensitisers, especially those which present positively charged moieties, allow the achievement of a strict control of the population of pathogens in waters of different origin. The main advantages of said sensitizers are as follows:
- a broad spectrum of action since an extensive drop in the population of a variety of microorganisms, such as Gram-positive and Gram-negative bacteria, a variety of fungal pathogens, viruses, and parasitic protozoa in both the cystic and vegetative stage, can be obtained by one irradiation protocol;
- the multi-target nature of the mode of action of photodynamic sensitisers makes it very unlikely that photoresistant strains of bacterial and fungal cells are selected;
- the overall phototreatment time is usually short owing to the short incubation required to achieve a significantly tight association of the photosensitizer with the microbial cells: actually, the binding is of electrostatic nature, namely, it is promoted by the real time interaction between the positively charged peripheral substituents in the photosensitiser molecule and the large number of negatively charged functional groups which are present in the outer wall of many microbial cells.

While a large number of polycyclic organic dyes can act as photodynamic sensitising agents (Boyle R.W. and Dolphin D., 2006, Structure and biodistribution relationships of photodynamic sensitisers, Photochem. Photobiol. 14: 4253-4259), porphyrins and their tetrapyrrole analogues, such as chlorins, porphycenes, phthalocyanines and naphthalocyanines, are generally recognized as the photosensitisers of choice especially for environmental applications involving aqueous systems. This statement is based on the following considerations:
- Porphyrins are characterized by a multi-band absorption spectrum ranging from the near-UV (360-400 nm) to the red (600-700 nm) spectral region. Therefore, porphyrins can be very efficiently photoexcited by essentially all the visible light wavelengths, opening the way to the use of intrinsically inexpensive lamps (e.g. fluorescent or filament tungsten lamps) emitting full spectrum visible light or even sunlight under specific geographical or climatic conditions.
- The most intense absorption band of porphyrins, the so-called Soret band, peaks in the 410-430 nm spectral region. Consequently, there is a very efficient interaction of the porphyrin photosensitiser with blue light wavelengths, which are endowed with maximum penetration power into natural waters out of the wavelengths which are present in the solar spectrum (Baker K.S. and Smith R.C., 1982, Spectral irradiance penetration into natural waters. In: The Role of Solar Radiation in Marine Ecosystems, ed. J. Calkins, 233-246. New York: Plenum Press).
- The flat aromatic macrocycle favours the partitioning of porphyrin derivatives in the lipid domains of cell membranes or their stable association with a variety of proteins; as a consequence, exogenously administered porphyrins have a high probability to be "intercepted" by specific sub-cellular sites before significant concentrations of the photosensitizing agent reach the genetic material. All the evidences available so far indicate that photoprocesses promoted by porphyrins have a very low mutagenic potential (Jori G. and Spikes J.D., 1994, Photobiochemistry of Proteins. In: Topics in Photomedicine, ed: K.C. Smith, 183-319. New York: Plenum Press). This feature is important for the safety of the procedure in the case of repetitive treatments.

A further advantage of the use of porphyrins as photodynamic sensitizers is that they can be modified by using a variety of synthetic strategies thus obtaining porphyrin derivatives whose physical and chemical properties, such as water-solubility, amphiphilicity, photo- and dark-stability, fine structure of the absorption spectrum, can be tailored to the specific properties of the system to be sterilized.

Several approaches for water disinfection using porphyrin-photosensitised processes have been carried out.

For example, the possibility of reducing the population of helminth eggs and faecal coliforms in wastewater through the combined action of the tetracationic porphyrin meso-(N,N,N,N-tetramethyl-pyridyl)porphine and irradiation with visible light or direct exposure to sunlight was described. By using micromolar porphyrin amounts and overall illumination times of about 4 hours, the coliform counts decreased by at least 5 logs (Alouini Z. and Jemli M., 2001, Destruction of helminth eggs by photosensitised porphyrin. J. Environ. Monit. 3: 548-551). Faecal coliforms and faecal enterococci also underwent an extensive mortality in wastewaters obtained from a sewage plant when the system was illuminated with full spectrum visible light in the presence of tetracationic porphyrins as the photosensitizing agents: about two logs of the usually very resistant Gram-negative faecal coliforms were inactivated upon addition of micromolar porphyrin concentrations and irradiation of the wastewater sample at a low fluence-rate, such as less than 10 mWcm⁻² (L. Costa et al., 2010, Sewage bacteriophage inactivation by cationic porphyrins: influence of light parameters, Photochem. Photobiol. Sci., 9: 1126-1134). The photosensitizing action of meso-substituted cationic porphyrins can also be utilized for obtaining an algicidal effect, as regards both the prevention of algae growth and the control of their proliferation; the latter process can be very important in some types of wastewater: both the planktonic cyanobacterium Planktothrix perornata (an odour-producing blue-green alga), which had been isolated from a water sample collected from a catfish pond, and a selected representative of green algae, namely Selenastrum capricornutum, underwent an about 50% growth inhibition upon illumination for 96 h with fluorescent light bulbs in the presence of micromolar amounts of a tetracationic meso-substituted porphyrin (Schrader K.V. et al., 2010, In vitro evaluation of the antimicrobial agent AquaFrin as a bactericide and selective algicide for use in channel catfish aquaculture, North Amer. J. Aquaculture, 72: 304-308). Lastly, the possibility to cure or prevent the often lethal infection by the fungus Saprolegnia parasitica in trout farming plants using a meso-substituted cationic porphyrin was demonstrated through field pilot studies (Magaraggia et al., 2006, *op. cit.).*

In spite of the encouraging results so far obtained in the field of the porphyrin-photosensitised disinfection of wastewater and water from fish-farming plants, serious problems are still associated with the direct introduction of the porphyrin into the medium to be disinfected.

First, the porphyrin itself could bind with selected constituents of the aqueous ecosystem to be disinfected and generate toxic effects, especially after long-term exposure; even more, the photogenerated highly reactive oxidizing species could attack vegetal or animal inhabitants of the aqueous medium and again cause various types of physical, chemical or biological alterations in the treated system.

Moreover, the porphyrin would be eventually spread into the environment and induce pollution or broadly diffused damages.

To improve the outcome of porphyrin-photosensitised water treatment, porphyrins (or analogues thereof) immobilized by covalent or affinity binding with water-swollen matrices have been prepared by chemical synthesis and tested *in vitro* for their antimicrobial efficiency.

Typical examples include meso-substituted p-aminophenyl-porphine and a peripherally substituted Zn(II)-phthalocyanine adsorbed on chitosan membranes (Bonnett et al., 2006, Water disinfection using photosensitisers immobilized on chitosan, Water Res. 40: 1269-1275). Along the same line, other inert matrices which can be readily associated with the photosensitizer have been proposed, including cellulose esters (M. Krouit et al., 2006, New photoantimicrobial films composed of porphyrinated lipophilic cellulose esters, Bioorg. Med. Chem. Lett., 16:1651-1655), agar surfaces (D. Caminos and E. Durantini, 2006, Photodynamic inactivation of Escherichia coli immobilized on agar surfaces by a tricationic porphyrin, Bioorg. Med. Chem. Lett., 14:4253-4259), cotton fabrics (C. Ringot et al., 2010, Amino porphyrin-grafted cotton fabric using 1,3,5-triazine link - application to antimicrobial phototherapy, Book of Abstracts International Congress Society of Porphyrins and Phthalocyanines, Albuquerque, USA, 2010, Abstract S26) or silicate matrices (S. Artrasky et al.,2006, Immobilization of zinc phthalocyanines in silicate matrices and investigation of their photobactericidal effect on Escherichia coli, Sci. World J., 6: 374-382).

US6107480 discloses porphyrin derivatives (metallated or unmetallated) covalently bound to polymers, to be used as photosensitizers for example in the treatment of waste waters. Porphyrins may be linked to the support material by an ester function.

WO2009006075 and WO2012059584 disclose porphyrins or derivatives thereof which can be immobilized and covalently linked on a solid support by methods known in the art. In particular, in WO2009006075 functionalized expanded porphyrins are described where a functional group is used to link the porphyrin to the solid support; the porphyrins are immobilized *via* covalent attachment to the solid support and they are used as spectrometric sensors for actinide cations.

CN101940948 discloses a method for immobilizing metalloporphyrins on a carrier by crosslinked polystyrene microspheres.

WO200138454 discloses metal-porphyrins to be used as catalyzers in the polymerization of organic contaminants. The catalyzer can be used immobilized on the surface of synthetic resins with a weak or covalent bond.

WO2003035553 discloses the use of phthalocyanine salts as photosterilising agents for microbiologically contaminated waters, which salts can be associated with an inert matrix.

WO0100322 discloses a photocatalyst which includes a photocatalytically active constituent which is immobilized on an ion-exchange resin, whose active constituent comprises a phthalocyanine and/or a tetraphenyl-porphyrin derivative.

EP984041 discloses phthalocyanine derivatives which may be covalently linked to a polymeric matrix.

WO199702963 discloses a tetrapyrrolic photosensitizer which may be covalently linked to a solid carrier, resin or particle, directly or by an inert organic spacer. Said organic spacer can be, for example, a hydrocarbon chain, a polylysine chain, a polyethylenglycol chain, a polysaccharide chain.

However, some important problems still remain unsolved in the art, such as the dark- or photo-toxicity of the porphyrin and the photogenerated intermediates for the ecosystems and the non-target organisms, as well as the tendency of porphyrin molecules bound in adjacent positions of the inert support to undergo aggregation due to the Π - Π interaction operating between the aromatic electron clouds above and below the tetrapyrrolic macrocycle; this leads to a drastic reduction of the photosensitising activity (Jori and Spikes, 1994, *op. cit*.). Lastly, the photoexcited porphyrin will undergo some degree of photobleaching, due to irreversible chemical modification of the molecule during irradiation (G. Jori and O. Coppellotti, 2007, Inactivation of pathogenic micro-organisms by photodynamic techniques: mechanistic aspects and perspective applications, Anti-infective Agents in Medicinal Chemistry, 6: 119-131). Also, the porphyrin macrocycle undergoes extensive modifications, including the formation of carbonyl type compounds, such as aldehydes or quinones; therefore, the consequent interaction between the biological organisms and the photochemically modified porphyrins could cause important damaging effects.

Therefore, there is still the need of a method and a system for the disinfection of water where porphyrins, or their derivatives, are immobilized on an inert matrix and which do not present the problems above described and unsolved in the prior art, in particular the drop in photosensitising efficiency due to photosensitiser aggregation, and the photo-toxicity and dark-toxicity generated by the porphyrin itself, its photodegradation products and the photogenerated reactive oxygen species.

### Summary of the invention

It has now been found that an adduct in which a porphyrinoid is bound to a resin through a specific spacer solves the problems above presented.

The use of a spacer between the resin and the porphyrinoid efficiently solves the problem of the drop of photosensitising efficiency due to photosensitiser aggregation. The flexibility thus imparted to the porphyrin molecule owing to the predetermined and properly selected distance from the resin surface facilitates the solvation of the photosensitiser by water and its energy transfer to oxygen in order to optimize the yield of hyper-reactive oxygen species and the intimate contact with the array of negatively charged groups at the outer surface of bacterial and fungal cells.

Also, the specific bonds established between the resin, the spacer and the porphyrinoid in the adduct of the invention minimize the risk of "dark" toxicity and, at the same time, allow for a simple and easy removal of the porphyrin and reconstitution of the adduct resin-spacer-porphyrinoid in case of photo-bleaching or degradation of one of the components.

It is therefore an object of the present invention, an adduct of general formula:

R-S-P

in which R is a resin, S is a spacer and P is a porphyrinoid, and wherein S is linked to R through a covalent bond and S is linked to P through an ionic bond.

The individual components of said adduct will be defined more in detail hereinafter in the description.

It is also an object of the present invention, a process for manufacturing said adduct comprising the steps of:
a) linking the spacer S to the resin R by adding an excess of the spacer in order to promote the formation of a covalent bond between the spacer and the resin;
b) adding an excess of the porphyrinoid P in order to form an ionic bond between the negative charges of the spacer S and the positive charges of the porphyrinoid P;
c) removing the unbound porphyrinoid P by repeated washing.

It is also an object of the present invention an apparatus comprising an irradiation chamber (1) provided with an inlet channel (2') for the entrance of water, wherein means for preventing passage of particulates (6') is placed at the point of entrance of the inlet channel (2') in the irradiation chamber, and an outlet channel (2") for water exit, wherein means for preventing passage of particulates (6") is placed at the point of exit of the outlet channel (2") from the irradiation chamber, at least one light source (3) positioned inside said irradiation chamber (1), and at least one resin-spacer-porphyrinoid adduct within said irradiation chamber (1).

The use of said apparatus for killing microbial species present in water, where for microbial species is intended microorganisms such as bacteria, fungi, viruses, parasitic protozoa in their vegetative and cystic stage, and algae, is a further object of the present invention.

It is also an object of the present invention, a system comprising said apparatus, in which disinfection of water takes place, a collector of the disinfected water, a hydraulic piping connecting the different elements, and a control panel.

The use of said system for disinfecting water from microbial species is also an object of the present invention.

### DESCRIPTION OF THE INVENTION

### Definitions

Within the context of the present invention, "photosensitizer" means a compound which absorbs electromagnetic radiation, preferably visible light, and is able to catalyse the formation of free radicals and/or singlet oxygen from triplet oxygen under the influence of the radiation. In the context of the present invention, the porphyrinoid compound is also named "photosensitizer".

Within the context of the present invention, "resin" means an inert support linked to the adduct spacer-porphyrinoid through a covalent chemical bond. In the context of the present invention, resin, matrix and solid support are used as synonyms. The resin can be of any nature, provided it is inert to the photochemical reaction of the photosensitizer. The resin can be inorganic or organic, the latter usually being a macromolecular compound.

Within the context of the present invention, "spacer" means a molecule having a first moiety which binds to the resin and a second moiety which binds to the porphyrinoid. "Spacer" is used in the present invention as a synonym of "linker". Its characteristics will be better disclosed hereinafter.

Within the context of the present invention, "adduct" means the product of a chemical reaction leading to the formation of an addition product between two or more chemical entities through the formation of one or more covalent bonds or stable interactions based on affinity forces.

Within the context of the present invention, "porphyrinoid" means a compound bearing a tetrapyrrole macrocycle. It includes, for example, porphyrins and their analogs.

### Figures

### DESCRIPTION OF THE DRAWINGS

Figure 1. Exemplary representation of a cylindrically shaped irradiation chamber (1), containing a number of plastic filters (4) filled with the resin-spacer-porphyrinoid adduct. The filters are irradiated by LED sources (3) characterized by a conical emission of light and placed on the walls of the irradiation chamber. An inlet channel (2') for water entrance and an outlet channel (2") for water exit are also shown. Mechanical filters are placed on the point of entrance (6') and of exit (6") of water to and from the irradiation chamber.
Figure 2. A: View from above of the LED distribution (3) along the walls of the irradiation chamber (1). B: Side view of a LED source fixed on the wall of the irradiation chamber and its conical light emission. C. Example of an embodiment of the present invention showing the arrangement of light sources (3) within a flexible tube (5) to be positioned in the irradiation chamber.
Figure 3. Expanded view of the micrometer-sized plastic filters filled with granules of the resin-spacer-porphyrinoid adduct (case A). Case B represents a different embodiment of the invention, wherein the resin-spacer-porphyrinoid adduct is deposited on the surface of a textile floating in the water.
Figure 4. Exemplary representation of a cylindrically shaped irradiation chamber, optionally equipped with a mechanical stirrer (not shown for sake of simplicity) and an air reservoir (9) from which air is bubbled into the water flowing across the chamber. The air reservoir (9) is connected to the chamber through channels (8); the air exits the chamber through the air outlet device (12), positioned on the top of the irradiation chamber. An air influx counteracting the water flow (dark arrows, 11) is thus generated.
Figure 5. Exemplary representation of a cylindrically shaped irradiation chamber, equipped with mechanical stirrers (7) and a number of deflectors (10) alternatively placed on opposite side of the walls of the irradiation chamber.
Figure 6. Typical representation of a system for the disinfection of microbiologically polluted water comprising the apparatus (irradiation chamber) of the invention and further elements.
Figure 7. Visible absorption spectrum of the CM-Sephadex-C1 porphyrin adduct suspended in PBS (a) and the supernatant obtained by centrifugation after 1 day incubation (b). C1 porphyrin concentration: 0.5 mg/ml.
Figure 8. Visible absorption spectrum of the CM-Sephadex-taurine-C1 porphyrin adduct suspended in PBS (a) and the supernatant obtained by centrifugation after 1 day incubation (b). C1 porphyrin concentration: 0.5 mg/ml.
Figure 9. Visible absorption spectrum of the CM-Sephadex-C1 porphyrin adduct suspended in PBS (a) and the supernatant obtained by centrifugation after 14 day incubation (b). C1 porphyrin concentration: 0.5 mg/ml.
Figure 10. Visible absorption spectrum of the CM-Sephadex-taurine-C1 porphyrin adduct suspended in PBS (a) and the supernatant obtained by centrifugation after 14 day incubation (b). C1 porphyrin concentration: 0.5 mg/ml.
Figure 11. Visible absorption spectrum of the CM-Sephadex-C1 porphyrin adduct suspended in PBS (a) and the supernatant obtained by centrifugation after 21 day incubation (b). C1 porphyrin concentration: 0.5 mg/ml.
Figure 12. Visible absorption spectrum of the CM-Sephadex-taurine-C1 porphyrin adduct suspended in PBS (a) and the supernatant obtained by centrifugation after 21 day incubation (b). C1 porphyrin concentration: 0.5 mg/ml.
Figure 13. Visible absorption spectrum in the visible wavelength interval of the supernatant obtained through centrifugation at 3,000g of an aqueous suspension of the samples CM Sephadex-C1 porphyrin (b) and CM Sephadex-taurine-C1 porphyrin (a) which had been incubated for 1 month in a neutral aqueous medium. C1 concentration: 0.5 mg/ml.

### Detailed description of the invention

### The resin-spacer-porphyrinoid adduct

The resin-spacer-porphyrinoid adduct is an object of the present invention.

Said adduct solves the technical problems and needs of the prior art discussed above and also provides the further advantages of being cheap and accessible.

The individual components of the adduct will be now described more in detail.

The resin is characterized by being readily solvated and/or swollen by water and chemically stable in an aqueous medium.

The resin is carboxylated with a -(CH₂)ₙCOOH group wherein n is comprised between 1 and 3.

Resins suitable for the present invention are, for example, selected from the group consisting of silica gel, cellulose, glass, polymers, including polyacrylamide gel beads or polystyrene-poly(ethylene glycol) graft, copolymer resins, and fiber optics.

The resin can be in a variety of sizes, shapes, and geometries, including flat surfaces, near-spherical beads, and thin fibers.

Suitable resins can be obtained from commercial sources or synthesized by methods known in the art.

Preferably, the resin is in the form of beads.

When the resin is in the form of beads, it can have different degrees of porosity. For example, the degree of porosity can range from 20 to 50 µm for the Sephadex G-25 derivatives and from 40 to 120 µm for the Sephadex G-75 derivatives.

Preferably, the resin is synthetically derived from hydrated silica or dextran polysaccharide.

More preferably, the resin is cellulose, Sephadex^{®} or Sepharose^{®}.

Cellulose is a molecule consisting of about 10,000 residues of glucose everyone carrying three reactive hydroxyl groups which form hydrogen bonds among adjacent chains thus packing the chains and/or allowing the addition of functional groups to increase water solubility. Sephadex^{®} is a trade-mark for cross-linked dextran gel, with functional ionic groups derivatized to generate superficial negative charges. Sepharose^{®} is a trade-name for a cross-linked polysaccharide polymer extracted from seaweed, bearing hydrophilic functional groups at the surface.

Said resins are commercially available or they can be synthesized by methods known in the art.

For example, when the resin is made of cellulose, a carboxymethyl cellulose (CMC) derivative is obtained; it improves water solubility and provides a potential binding site for additional functional groups (e.g. amino groups) by very stable covalent bonds.

The spacer is a bi-dentate compound comprising a first moiety which covalently binds to the resin and a second moiety negatively charged which establishes a stable ionic link with the positively charged moieties of the porphyrin in the adduct.

The spacer S has the following general formula:

A-X-B

wherein A is a moiety which covalently binds to the resin and it is selected from the group consisting of an amino group, an alcohol group and a thiol group,
X is (CH₂)_{z} or (O-CH₂-CH₂)_{y} wherein z is comprised between 1 and 20 and y is comprised between 1 and 50,
and B is a negatively charged moiety which establishes a ionic link with the positively charged moieties of the porphyrinoid P of the adduct.

Preferably, A is an amino group.

Preferably, B is a carboxylate, sulphonate or phenolate group.

X can be chosen according to the degree of motility and hydrophilicity desired for the terminal porphyrinoid molecule (P).

In a preferred embodiment, the spacer is taurine (2-amino-ethansulphonic acid), wherein A is an amino group, B is a sulphonate group, X is (CH₂)₂. The sulphonate group remains fully dissociated, hence negatively charged, even in moderately acidic water media as a consequence of the high level of acid strength typical of sulphonic acids.

The flexibility acquired by the porphyrinoid molecule and the presence of a determined distance between the porphyrin and the resin, thanks to the presence of the spacer of a selected length, allows for the following advantages:
a) the solvation of the porphyrinoid by water, thereby reducing the intensity of the Π - Π attractive interaction between adjacent porphyrinoid moieties and minimizing the tendency to generate aggregated porphyrin clusters;
b) the energy transfer from the electronically excited photosensitiser to oxygen thanks to the easier accessibility of the porphyrinoid, thereby optimizing the yield of hyper-reactive oxygen species;
c) the intimate contact of the photosensitiser with the array of negatively charged groups at the outer surface of bacterial and fungal cells, thus enhancing the rate and probability of photoinduced damage to the constituents of the microbial cell.

The component P of the adduct of the invention is a porphyrinoid selected from the group consisting of: porphyrin, reduced porphyrin, isomer of porphyrin, phthalocyanine and naphthalocyanine.

When P is a porphyrin, it has the following general formula (I): 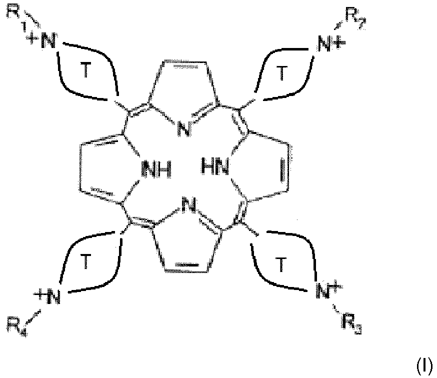 wherein:
T is an heterocyclic ring selected from the group consisting of pyridyl, piperidyl and pyrimidyl; R₁=R₂=R₃ is -CH₃, and
R₄ is selected from the group consisting of straight or branched, saturated or unsaturated C₁-C₂₂ hydrocarbon chain; C₆-C₁₀ aromatic, C₆-C₁₀ heteroaromatic ring containing one or more heteroatoms selected from the group consisting of oxygen, sulphur and nitrogen, and/or mixed C₁-C₂₂ alkyl - C₆-C₁₀ aromatic moieties.

Preferably, T is a pyridyl.

Preferably, R₄ is a straight or branched, saturated or unsaturated, C₁-C₂₂ alkyl chain.

More preferably, R₄ is selected from the group consisting of: -CH₃, -CH₂(CH₂)₄CH₃; -CH₂(CH₂)₈CH₃;-CH₂(CH₂)₁₀CH₃; -CH₂(CH₂)₁₂CH₃; -CH₂(CH₂)₁₆CH₃ or -CH₂(CH₂)₂₀CH₃.

Most preferably, R₄ is -CH₃, -CH₂(CH₂)₁₀CH₃ or -CH₂(CH₂)₁₂CH₃.

The invention includes within its scope all the possible stereoisomers, Z and E isomers, optical isomers and their mixtures of compounds of formula (I).

Preferred porphyrins are:
meso-tetra(N,N,N,N-methyl-pyridyl)porphine (C1);
meso-tri(N-methyl-pyridyl), mono(N-dodecyl-pyridyl)porphine (C12);
and meso-tri(N-methyl-pyridyl), mono(N-tetradecyl-pyridyl)porphine (C14).

In general, for water disinfection, meso-substituted porphyrins are preferentially adopted. A meso-substituted porphyrin is a porphyrin comprising one to four quaternarized nitrogens in the meso positions of the tetrapyrrole core, where the positive charge is generated by binding of the nitrogen atom through its lone electron pair with hydrocarbon chains of length ranging from 1 to 20 carbon atoms: such meso mono-to tetra-substituted porphyrins have been shown to be endowed with a marked phototoxic activity against both Gram-positive and Gram-negative bacteria, including antibiotic-resistant strains (E. Reddi et al., 2002, Photophysical properties and antibacterial activity of meso-substituted cationic porphyrins, Photochem. Photobiol. 75: 462-470). Antibacterial photoproperties of meso-tetra(N-alkyl-pyridyl)porphines have been studied in detail, even though optimal photosensitizing effects on microbial cells have been also obtained by replacement of the pyridine ring with piperidine or morpholine (Magaraggia et al., 2006, *op. cit*.; D.A. Caminos et al., 2006, Photodynamic inactivation of Escherichia coli by novel meso-substituted porphyrins, by 4-(3-N,N,N-trimethylammoniumpropoxy)phenyl and 4-(trifluoromethyl)phenyl groups, Photochem. Photobiol. Sci., 5:56-65).

Meso-tri(N-methyl-pyridyl), mono(N-dodecyl-pyridyl)porphine (C12) and its mono-N-tetradecyl analogue (C14) have been recently proposed as particularly efficacious photosensitizers for water disinfection (J.C. Bommer and G. Jori, 2003, Photodynamic porphyrin antimicrobial agents, US Patent 6,573,258). The molecule of these porphyrins has been carefully engineered so that the multi-carbon dodecyl or tetradecyl chain penetrates into the lipid domains of cell membranes in order to anchor the porphyrin to the membrane itself and alter the three-dimensional organization of the outer wall typical of many types of microbial cells with a consequent enhancement of the membrane permeability; at the same time, the four positive charges interact electrostatically with the anionic surface of microbial cells.

All the compounds used in the present invention are well-known in the chemical literature and/or commercially available. Reference can be made to the general literature, see for example Kadish, Smith and Guillard (eds), The Porphyrin Handbook, Academic Press.

When P is a reduced porphyrin, it is selected from the group consisting of chlorin and bacteriochlorin. When the porphyrin is reduced by hydrogenation of one carbon-carbon double bond in a pyrrole ring, a chlorin is originated; when two carbon-carbon double bonds in two adjacent pyrrole rings are reduced in the porphyrin, a bacteriochlorin is originated. Structures of chlorin and bacteriochlorin are well known in the art.

When P is an isomer of porphyrin, it is a porphycene, which is the 18Π electronic isomer of a porphyrin.

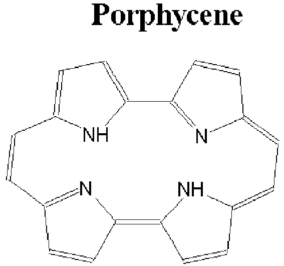

In another embodiment, P is a phthalocyanine or a naphthalocyanine. When the four pyrrole rings of the porphyrin are fused with a benzene ring, a phthalocyanine is obtained. When the four pyrrole rings of the porphyrin are fused with a naphthalene ring, a naphthalocyanine is obtained.

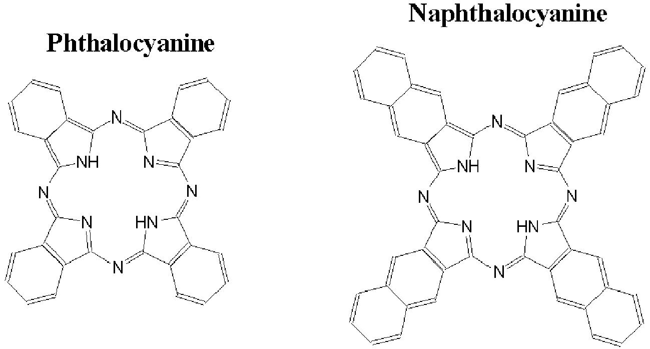

The component P can further contain a chelated metal ion, which does not impair the photodynamic activity by shortening the lifetime of the electronically excited states. Said metal ion is selected from the group consisting of: Mg(II), Zn(II), AI(III), In(IV), Ge(IV), Si(IV), Sn(IV), Pd(II).

The extensive photosensitised inactivation of the microbial species when in contact with the adduct of the present invention is a consequence of the tight interaction of the cationic porphyrinoid with the array of negatively charged moieties, for example carboxylate groups from teichoic and lipoteichoic acids, which are present at the outer surface of bacterial cells. The close proximity between the photoexcited photosensitiser and the photosensitive targets which stabilize the tight three-dimensional architecture of the cell outer wall causes an increase in the permeability of such structure and facilitates the penetration of the photosensitiser to the inner cellular compartments, whose integrity is critical for cell survival (T. Maisch, 2009, A new strategy to destroy antibiotic resistant microorganisms: antimicrobial photodynamic treatment, Mini-review Med. Chem., 9: 947-983).

An advantage of the adduct of the invention is that the binding of the porphyrinoid to the resin through the spacer of the invention minimizes the risk of "dark" toxicity since the bond between the strong acidic functional group of the spacer and the cationic groups in the porphyrin molecule is more stable than the weaker direct binding between the resin and the porphyrinoid.

The resin-spacer-porphyrinoid adduct of the invention has the further advantage that it can be reutilized for several cycles of water photodisinfection, as a consequence of the long-term stability of the system, even when it is suspended in the aqueous medium, and of the essentially negligible degree of photobleaching when the system is exposed to full spectrum visible light.

Furthermore, the non-covalent bond established between the tetracationic porphyrinoid or derivatives thereof and the negatively charged spacer allows for a fast replacement of any partially or even extensively damaged porphyrinoid; the latter can be readily removed by easily available chemical approaches (e.g., washing with hydrochloric acid) and addition of new porphyrinoid stoichiometrically equivalent to the amount of porphyrin removed. For the same reason, if the resin or the spacer is degraded, the porphyrinoid can be easily separated from the adduct and a new one can be quickly restored.

### The apparatus

The resin-spacer-porphyrinoid adduct can be included in an apparatus.

The functioning of the apparatus is based on the principle of flow reactors, namely the water is disinfected by a dynamic approach while flowing across the irradiation chamber.

The apparatus comprises an irradiation chamber (1) provided with an inlet channel (2') for the entrance of water, wherein means (6') for preventing contaminating materials entering and the beads of the resin-spacer-porphyrinoid adduct exiting is placed at the point of entrance of the inlet channel (2') in the irradiation chamber, and an outlet channel (2") for water exit, wherein means (6") for preventing passage of particulates is placed at the point of exit of the outlet channel (2") from the irradiation chamber, at least one light source (3) positioned inside said irradiation chamber (1), and at least one resin-spacer-porphyrinoid adduct within said irradiation chamber (1).

The components of the apparatus, which is an object of the present invention, will be now described in detail.

The irradiation chamber (1) can be in any convenient shape, conical, cylindrical or rectangular shape and it can develop vertically or horizontally.

The size of the irradiation chamber is designed according to the required capacity, volume and flow of the microbiologically polluted water. For example, if both the input and output of water are 10 litres per min. and the time for achieving the required (>3 log) level of disinfection is 10 min., the irradiation chamber is sized to contain 100 litres of water.

The irradiation chamber (1) is generally connected with the place where the disinfected water needs to be used.

For example, but not exclusively, it can be connected with a fish-farming tank, a fish egg incubator, a container of waste water of industrial origin, a reservoir for irrigation or potable water, a swimming pool.

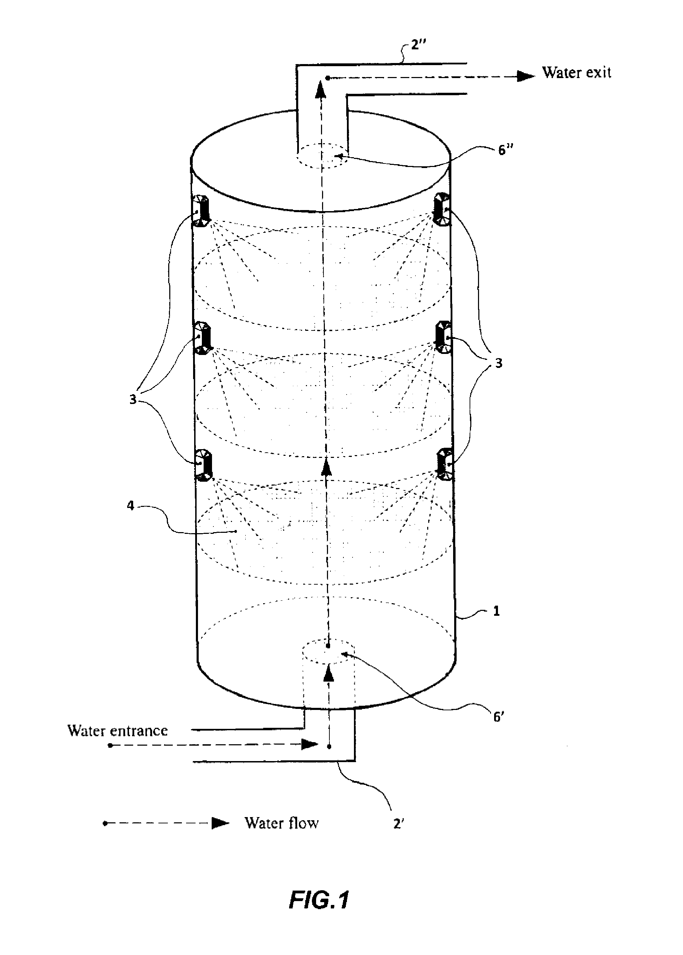

Means (6') for preventing passage of large size impurities, for example a mechanical filter, is placed at the point of entrance of the inlet channel (2') into the irradiation chamber (see for example Figure 1). The role of said means is to stop any gross impurity or large particles which could increase the turbidity of water, deviate the light beams used to excite the porphyrin photosensitiser, and damage the resin-spacer-porphyrinoid adduct. When said means is a mechanical filter, the diameter of the pores of said filter is preferably between 1 and 2 mm.

Means (6") for preventing passage of particulates, for example a mechanical filter, is also placed at the point of exit of the outlet channel (2") from the irradiation chamber to avoid the release of the resin-spacer-porphyrinoid adduct out of the irradiation chamber (see for example figure 1). For example, said means can be a mechanical filter and if the resin of the adduct R-S-P is in the form of beads, said filter has pores of dimensions smaller than the size of the resin beads of the adduct. Said mechanical filter is preferably made of plastic.

The at least one light source (3) is positioned inside the irradiation chamber (1).

Preferably, it is fixed on the inside walls of the irradiation chamber, at predetermined intervals, thus allowing the obtainment of a maximally uniform illumination of the R-S-P adduct.

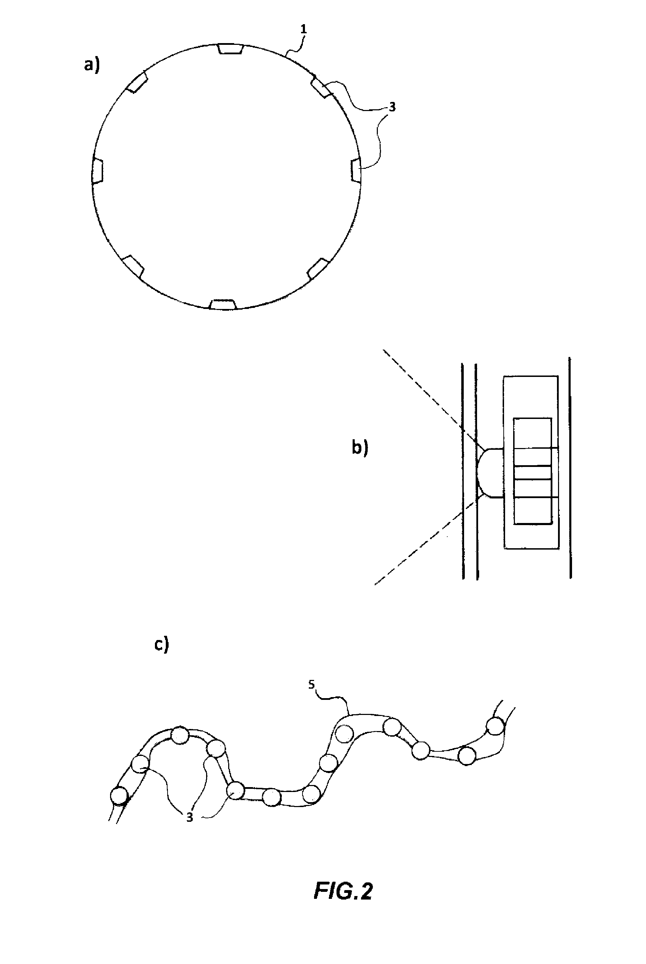

In an alternative embodiment, the light source is included in transparent tubes (5) fixed on the inside walls of the irradiation chamber, as it is schematically represented in Figure 2c.

Preferably, there is more than one light source inside the irradiation chamber.

The light source is used to electronically excite the porphyrinoid of the R-S-P adduct.

A suitable light source can be selected from the group consisting of: a light-emitting diode (LED), a filament lamp, a fluorescent lamp, and a high pressure xenon or mercury lamp. When the light source is a high pressure xenon or mercury lamp, the UV emission is blocked by the insertion of cut-off filters in the optical path.

Preferably, the light source is a LED.

LEDs are characterized by an emission spectrum involving a restricted wavelength interval, namely ca. 20 nm at half emission band.

Preferably, the LED matches the 420 nm peak of the main absorption band of porphyrins and their analogues: such wavelength is intensely absorbed by porphyrins which exhibit a molar extinction coefficient higher than 100,000 M⁻¹cm⁻¹. The use of said LED allows for the use of smaller porphyrin doses to reach a given threshold of microbial inactivation; moreover, the blue light wavelengths are endowed with a maximum penetration power into water (the 1/e or 37% value of the incident light intensity is reached at a distance of 20 m from the aqueous surface, see G. Jori, E. Reddi, 1991, Second generation photosensitizers for the photodynamic therapy. In: Doughlas R, et al., eds. Light in Biology and Medicine, pp 253-266. London: Plenum Press.), thereby allowing the obtainment of a uniform illumination of large water volumes.

A further advantage of the use of LED is that its narrow emission range guarantees a precise dosimetry of the incident light, which leads to the definition of irradiation protocols closely tailored to the characteristics of the aqueous medium to be disinfected with significant advances as regards both the efficiency of the operation and energy saving. The absence of the infrared and ultraviolet components in the LED emission spectrum eliminates the need of safety measures for the operators, the users and the ecosystem, as well as minimize the probability of thermally induced undesired side effects, due to water heating.

Finally, LEDs are low cost devices and generally display a long (>1,000 hours) duration of their emission intensity.

The at least one resin-spacer-porphyrinoid adduct located inside the irradiation chamber can be included in a filter, loaded on a tissue or dispersed.

In one embodiment, the R-S-P adduct is included in a filter (4). Namely, the filter comprises a superior layer and an inferior layer and the R-S-P adduct is included between the two layers of the filter. In said embodiment, the irradiation chamber further comprises one or more filters (4), see for example figure 1. Said filter is made of a material transparent to visible light, preferably a plastic material. The filter is endowed with a high mechanical resistance to the pressure exerted by flowing water. The filter has pores; when the resin R is in the form of beads, the dimensions of said pores are smaller than the size of the resin beads but large enough to allow a sufficiently high rate of water flow with an acceptable pressure. Preferably, the diameter of said pores ranges from 10 to 20 µm, which is smaller than the size of the beads forming the Sephadex, Sepharose or cellulose resins (generally, the size of their wetted granules are in the >150 µm range).

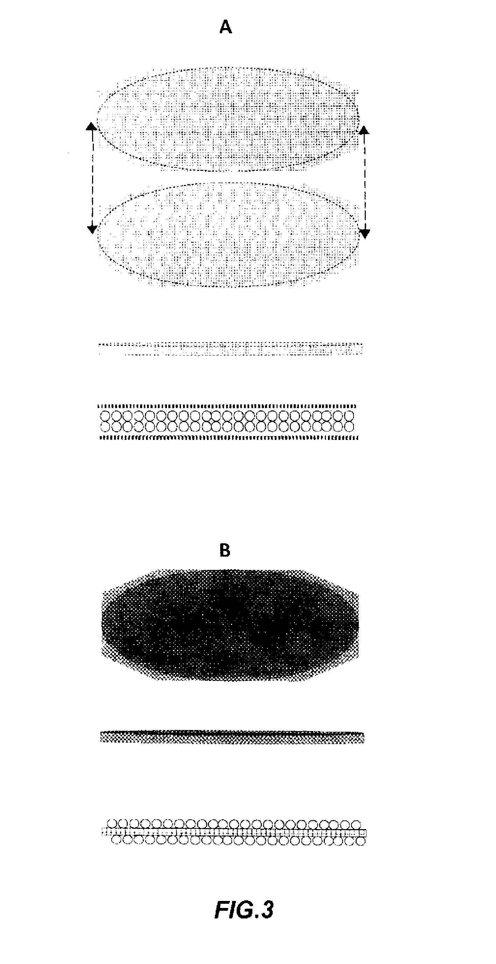

In a preferred embodiment, the filters (4) are fixed at the inside walls of the irradiation chamber and are placed in parallel between them at a predetermined distance, orthogonally to the direction of water flow (see Figure 1). Each filter comprises one or more layers of the resin-spacer-porphyrinoid adduct. The total thickness of the filter is preferably no more than 5 mm. Preferably, the R-S-P adduct included in the filter is in the form of granules (Figure 3, case A). The upper and lower surfaces of each filter are illuminated by the light sources (3), which are placed in between each two filters and orientated in such a way to obtain a uniform irradiation of the porphyrinoid inside each filter thanks to their conically-shaped light emission.

When the light source(s) is(are) included in transparent flexible tubes (5) fixed on the walls of the irradiation chamber (as in Figure 2c), said tubes can be located between each two filters, so that each filter and the included R-S-P adduct are uniformly and simultaneously illuminated from both below and above.

In this embodiment, water is pushed by a pump and it enters into the irradiation chamber from the bottom through the inlet channel (2'), it flows upwards crossing the various filters (4) comprising the resin-spacer-porphyrinoid adduct, and it eventually exits from the irradiation chamber (3) through the outlet channel (2") (Figure 1). When the water flows through the filters, it undergoes a number of sequential steps of porphyrin photoexcitation and microbial cell inactivation.

The water flow rate is modulated to favour a high level of water oxygenation and minimize the formation of light-scattering air bubbles.

In a different embodiment, the at least one resin-spacer-porphyrinoid adduct is loaded by affinity binding on a tissue, for example wool, which is readily impregnated by water (Figure 3, case B). Said tissue floats in the water, impregnated with the spacer-porphyrinoid adduct, and guarantees an efficient interaction between the porphyrinoid and pathogenic cells polluting the water.

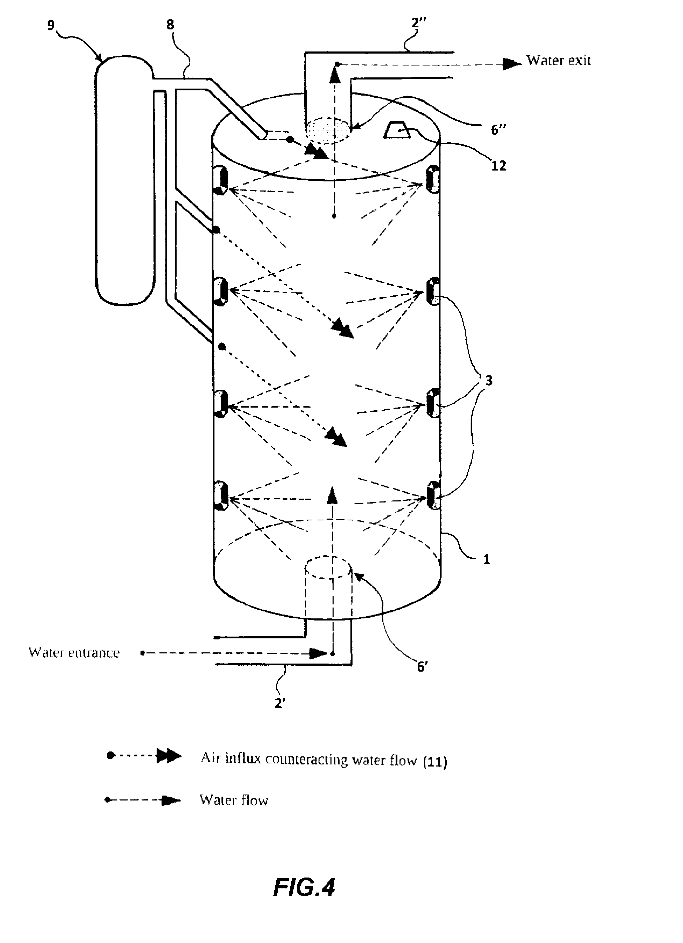

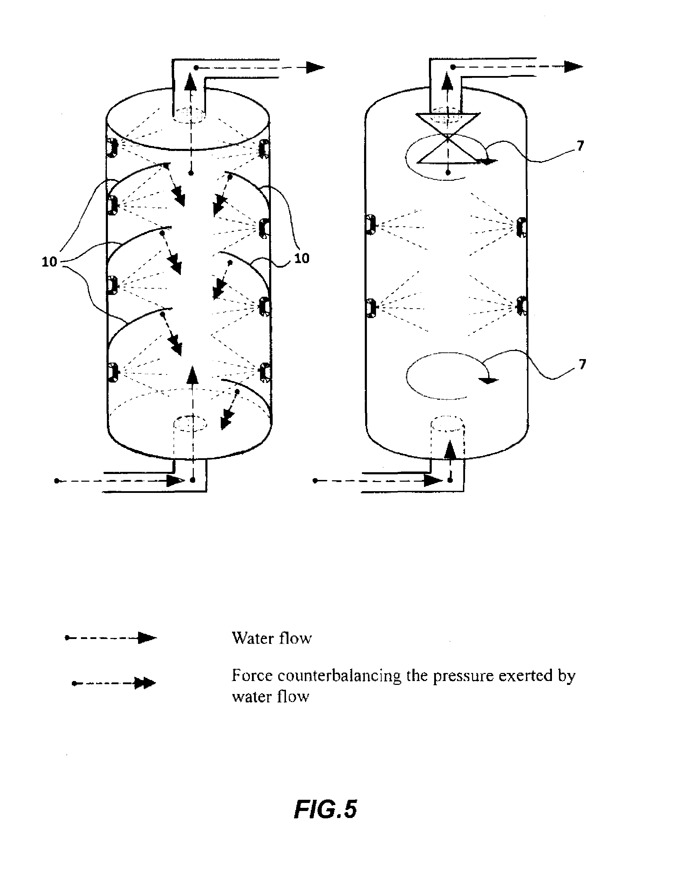

In another embodiment of the invention, the resin-spacer-porphyrinoid adduct is dispersed inside the irradiation chamber (1). In this embodiment, the adduct is allowed to freely float in the water medium entering the irradiation chamber through the inlet channel (2') and crossing all the chamber. A schematic representation of this embodiment of the invention is shown in Figures 4 and 5, where different elements of the apparatus are shown. The water moving across the chamber is exposed to an influx of air (11) to further stir the water and at the same time guarantee a steady supply of oxygen, that is the main cytotoxic agent involved in the photosensitised process. The air enters the irradiation chamber (1) through channels (8) which connect the irradiation chamber with a compressed air supplier (9), as shown in figure 4. Also, an air outlet device (12) is positioned on the top of the irradiation chamber, allowing the exit of the air. To avoid the accumulation of the adduct in specific sites of the chamber (e.g. at the walls of the chamber) as a consequence of the pressure exerted by the water flow, the water is continuously mixed by means of mechanical stirrers (7) counterbalancing the direction of the water flow, as shown in figure 5. Also, to slow down the rate of water flow and favour a uniform distribution of the resin-spacer-porphyrinoid adduct throughout the irradiation chamber (1), one or more deflectors (10) are disposed on opposite inside walls of the chamber (as shown in Figure 5). The light sources (3) are placed along the inside walls of the irradiation chamber and operated at a light fluence rate which achieves a full illumination of the aqueous medium. Preferably, the resin-spacer-porphyrinoid adduct is dispersed homogeneously inside the irradiation chamber (1).

### The system

The apparatus of the invention can be used in a system for the disinfection of microbiologically polluted water.

The system, which is also an object of the present invention, comprises the apparatus of the invention as above described, a collector of the disinfected water, a hydraulic piping connecting the different elements and a control panel.

The disinfection of water takes place in the apparatus.

In one embodiment, the system further comprises a buffering tank, wherein water is collected before flowing into the apparatus.

Optionally, the system can also comprise one or more of the following elements: manometer, flowmeter, hydraulic valves, centrifugal pump, frequency inverter.

The water can be recycled from the buffering tank back to the buffering tank (or from the apparatus to the apparatus when the buffering tank is not present), until the desired level of disinfection is achieved.

When the desired level of disinfection is achieved, the disinfected water is collected into the collector.

A water sample, for example of about 5 milliliters, is shifted into the control panel for a real time continuous monitoring of the efficiency of the process; said monitoring involves the analysis of the water sample by means of a colorimetric assay via the "live/dead kit", which is based on the differential dyeing of surviving or dead microbial cells and subsequent spectrophotometric measurement of the colour intensity (T. Maisch et al., 2004, Antibacterial photodynamic therapy in dermatology. Photochem Photobiol Sci 3: 907-917).

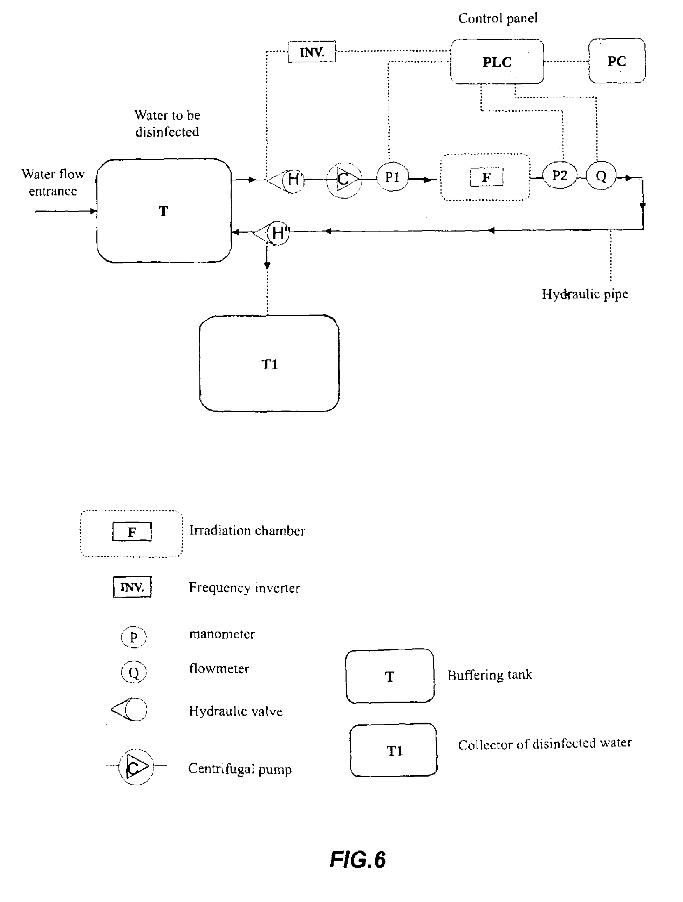

A non limiting example of the system of the invention is represented in the scheme of figure 6.

An example of functioning of the system of the invention, with reference to the scheme of figure 6, is as following.

The water to be disinfected is pushed from the buffering tank (T) into the apparatus/irradiation chamber (F) thanks to the action of a pump (C); the water pressure at the entrance and exit of the irradiation chamber is monitored by manometers placed before (P1) and after (P2) the apparatus F. At the same time, the rate of water flow is controlled by a flowmeter (Q), generally placed after the irradiation chamber. The water is then driven to the final collector (T1) via a standard hydraulic pipe. A frequency inverter (INV) is connected with the hydraulic valve (H') placed between the buffering tank (T) and the irradiation chamber (F) and allows to adjust the water flow on the basis of the indications provided by the flowmeter (Q). The programmable logic controller (PLC, or control panel) is the electronic interface to which the individual elements, such as the manometers (P) and the flowmeter (Q), are connected and is in turn controlled via software by a computer (PC); this panel is used by the operator to fine tune the values of the various parameters involved in the overall process of water disinfection, as well as to evaluate in real time the efficacy of the inactivation of the pathogens through the "dead/live kit".

On the basis of the values for parameters Q and P, hence the total size of F, the timing for achieving a satisfactory degree of decrease in the microbial charge can range from real time to time corresponding with an at least 3 log decrease in the population of the microbial pathogens.

Therefore, parameters Q and P shall be chosen in order to achieve the desired water disinfection degree, wherein a satisfactory disinfection degree corresponds to an at least 3 log decrease in the population of the microbial pathogens.

### Uses

It is an object of the present invention the use of the apparatus of the invention to kill microbial species present in water, wherein for microbial species is intended microorganisms such as bacteria, fungi, parasitic protozoa in their vegetative and cystic stage, viruses and algae.

It is also an object of the present invention, the use of the system of the invention for disinfecting water from microbial species.

The present invention will be now further described by means of non-limiting examples.

### Example 1

### Studies with CM-Sephadex-porphyrin systems with and without the spacer

### Spectroscopic studies

Initial studies about the antimicrobial activity of visible light-excited resin-spacer-porphyrin systems were carried out by using CM-Sephadex-taurine-C1 porphyrin adducts, where C1 indicates the meso-(N,N,N,N-tetra-methyl-pyridyl)porphine.

The C1 porphyrin was obtained as a commercial product from Sigma Chemical Co., in the form of a crystalline powder with a 99% purity. Other meso-substituted porphyrins were prepared by chemical synthesis according to the procedure described by Bommer and Jori in the US Patent 6,573,258 (2003). The direct attachment of the cationic porphyrins to the CM-Sephadex resin was obtained by formation of an ionic bond between the negatively charged carboxylate moieties in the Sephadex and the four positive charges in the porphyrin molecule. Typically, the CM-Sephadex (1 g) was incubated at room temperature with a ten-fold molar excess of C1 in water buffered at pH 8.5. The aqueous suspension was kept for 3 h under gentle magnetic stirring. At the end, any unbound porphyrin was removed by repeated washing with phosphate-buffered saline until no additionally removed porphyrin could be detected by spectrophotofluorimetric analysis of the washing water (excitation at 420 nm, observation of the emission at 660 nm). On the average a 24% loading of the CM-Sephadex by the porphyrin was found to occur. The covalent binding of taurine to the CM-Sephadex was obtained by adding a 10-fold molar excess of the amino acid to the aqueous medium buffered at pH 8.0 in order to promote the classical reaction for the formation of amides from activated carboxylic acids; then, a 10-fold molar excess over taurine of the C1 porphyrin was added and the resulting suspension was stirred for 3 h at room temperature. The unbound porphyrin was removed by repeated washing as above described: an about 60% loading with C1 was obtained. The spectroscopic and photosensitising properties of this adduct were compared with those typical of the spacer-free CM-Sephadex-C1 porphyrin adduct.

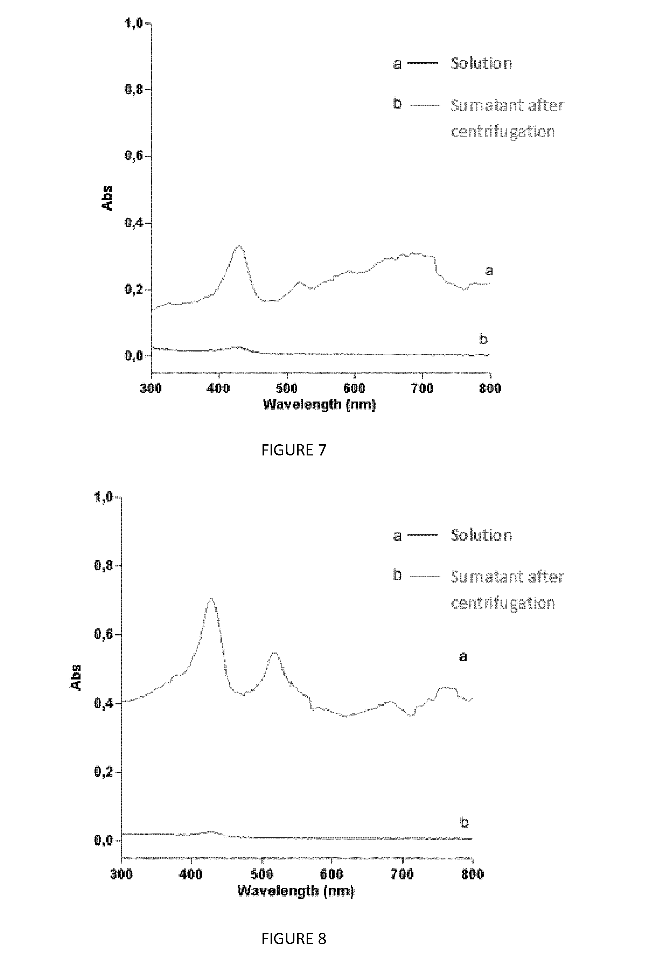

Upon suspension of the two samples in an aqueous solution of phosphate-buffered saline (PBS) at neutral pH 7.4, the absorption spectrum of the CM-Sephadex-C1 porphyrin adduct in the visible wavelength range (Figure 7) showed distinct variations as compared with the absorption spectrum of its taurine-containing analogue (Figure 8). Thus, the total optical density of the most intense absorption band, namely the so called Soret band peaking at 422 nm, was found to be 0.11 for the taurine-free derivative and 0.15 for the taurine-containing derivative, after correction for subtracting the contribution to the total optical density by scattering; moreover, the half-band width of the absorption spectrum was equal to 0.51 and 0.33 nm for the taurine-free and taurine-containing derivative, respectively. The adduct with taurine was also characterized by a better resolution of the less intense band at 520 nm, typical of meso-substituted porphyrins. These features, namely hypochromicity, broadening and poorer resolution of the absorption bands, as observed for the CM Sephadex-C1 adduct, are typical of aggregated porphyrins (J.H. Furhop and K.M. Smith,1975, Electronic absorption spectra of porphyrins, in: ed. K.M. Smith, Porphyrins and Metalloporphyrins, pp.871-889, Elsevier: Amsterdam), a process often occurring for porphyrins in aqueous media as a consequence of the strong π-π interactions established between the aromatic electron clouds of the tetrapyrrolic macrocycle for adjacent porphyrin molecules (R.W. Boyle and D. Dolphin, *op. cit.).* This tendency is weakened by the presence of the taurine spacer, which increases the degree of freedom for the porphyrins allowing for greater solvation by water and longer average distance between the resin-bound tetrapyrrolic moieties. The markedly more extensive monomeric state for the porphyrin bound to the CM-Sephadex-taurine system is of utmost importance as regards the photosensitising efficiency. It is well known (E. Reddi et al., *op. cit*.) that aggregation shortens the lifetime of the electronically excited states of porphyrin oligomers owing to an enhanced probability of non-radiative decay to the ground state, thereby decreasing the probability of excited porphyrin interaction with oxygen to generate the cytocidal reactive oxygen species. In actual fact, the quantum yield for singlet oxygen production was measured for the two samples using the molecular probe disodium 9,10-anthracene dipropionic acid (ADPA): in the presence of singlet oxygen, the ADPA is photobleached through its conversion to the endoperoxide, leading to a reduction in the intensity of the absorption bands of the probe (G. Jori and J.D. Spikes, *op. cit*.). The quantum yield value decreased from 0.41 for the taurine-loaded sample to 0.29 for the taurine-devoid analogue.

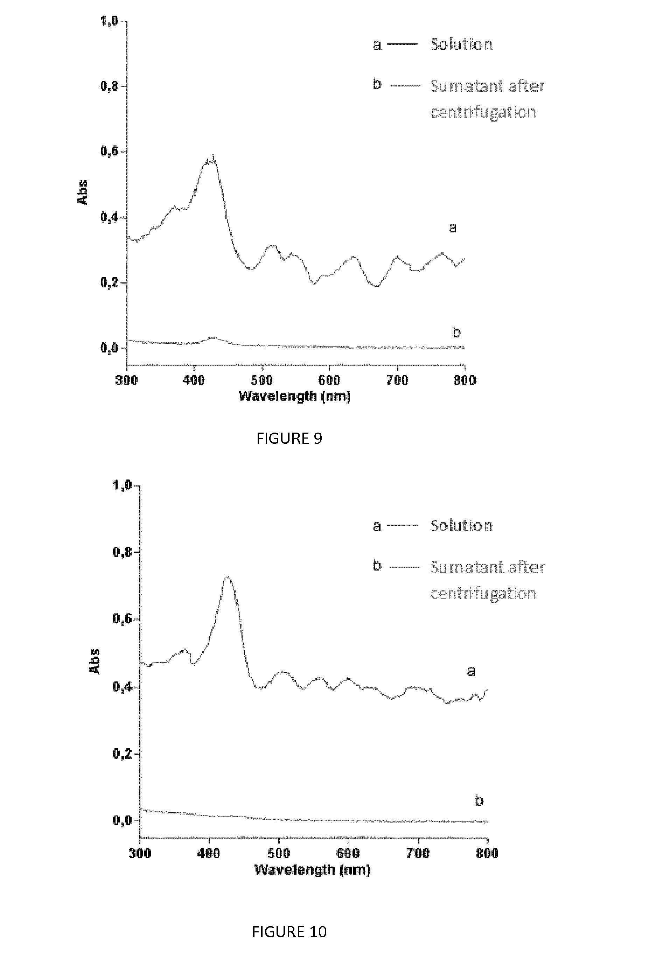

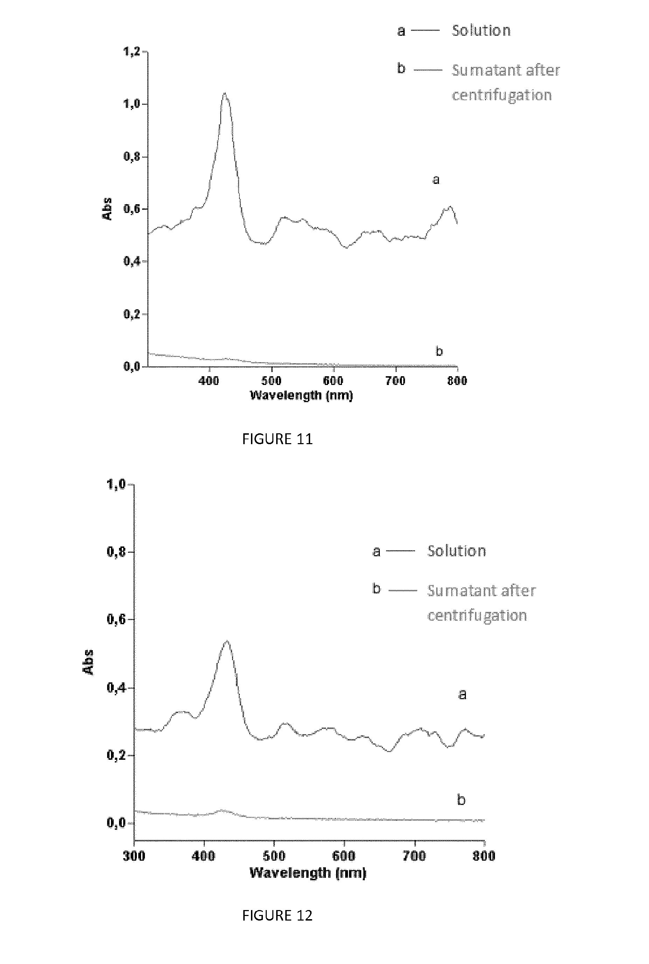

A comparison between the visible absorption spectra of the CM Sephadex-C1 porphyrin adduct and its taurinated analogue taken after 14 days of incubation in water (PBS, pH 7.4), as shown in Figure 9 and, respectively, Figure 10, demonstrates that the presence of taurine stabilizes the monomeric state of the C1 porphyrin in the aqueous medium since the optical density of the porphyrin at the maximum of the Soret band and the half-band width are essentially identical with the values measured at day 1, whereas the sample devoid of taurine displays an increased enlargement of the Soret band with the additional appearance of a shoulder around 380 nm, which is suggestive of the formation of higher order aggregated porphyrin species. The splitting of the Soret band into two distinct bands (higher and lower order oligomers of C1 porphyrin) is even more evident in the spectra obtained for the CM-Sephadex-C1 adduct on day 21 (Figure 11), while the Soret band of the CM Sephadex-taurine-C1 porphyrin (Figure 12) still exhibits the features typical of monomeric porphyrins in spite of the larger concentration used, which should favour the formation of aggregated species.

Quite importantly, the analysis of the absorption spectra points out that there is no appreciable cleavage of the strong ionic bond between the anionic sulphonate group of taurine and the positively charged groups of the C1 porphyrin, at least during a continuous incubation of the adduct in neutral water for up to three weeks: Figures 8, 9 and 11 show no detectable presence of free porphyrin in the supernatant obtained after centrifugation of the water where the CM Sephadex-taurine-C1 porphyrin had been incubated; the centrifugation had been performed in order to precipitate and filter out the resin and the associated porphyrin.

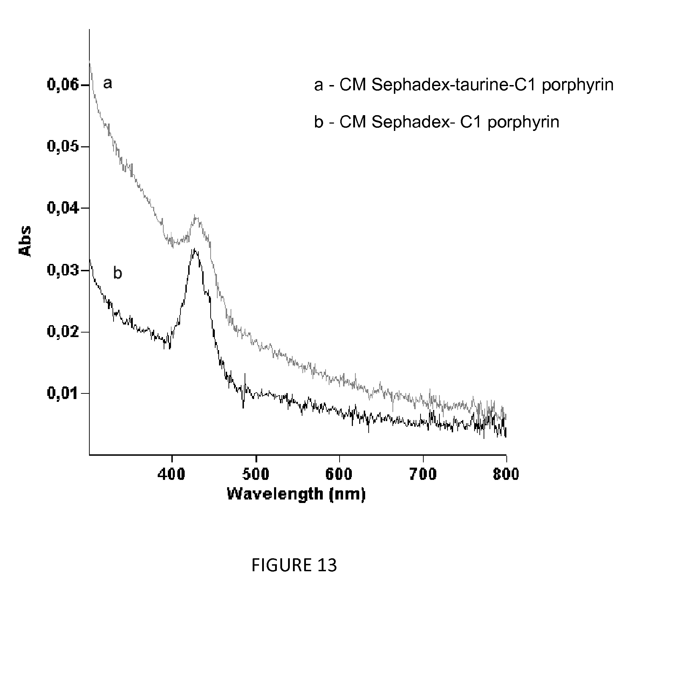

The release of the C1 porphyrin into the aqueous medium begins to be detectable only after incubation of the adducts in PBS for 1 month, since for both samples a clearly visible absorption band peaking at 422 nm appears in the spectra of the supernatants isolated by centrifugation of the resin suspensions (Figure 13). It is evident that the intensity of the peak recorded for the porphyrin isolated from the CM-Sephadex-C1 porphyrin adduct is about three times larger than the intensity of the peak recorded for the CM Sephadex-taurine-C1 porphyrin adduct; this observation further underlines the greater stability of the porphyrin associated with the CM Sephadex-taurine system.

A different result was found to occur when the CM Sephadex-C1 porphyrin and the CM-Sephadex-taurine-C1 porphyrin adducts were tested for their stability in an acidic aqueous medium (namely, 1 M HCl) to simulate the situation often occurring for waters present in industrialized areas. Under these conditions, the porphyrin directly associated with the CM Sephadex was found by spectrophotometric analysis to be present in a purely monomeric state, while its bond with the resin was totally split at one day after the suspension of the adduct in the acid aqueous medium: thus, after centrifugation of the suspension of the adduct, all the porphyrin was recovered in the supernatant. On the other hand, in the presence of the taurine spacer the C1 porphyrin was only partially released even in such a drastic acidic environment after incubation for one day, since a significant fraction of the porphyrin (about 50%) was still associated with the resin-taurine adduct, as shown by spectrophotometric analysis. A complete removal of the porphyrin from the CM Sephadex-taurine adduct was obtained only after incubation in HCl 1 M for 3 days.

### Example 2

### Photosensitised inactivation of bacteria

The efficiency of photoactivated CM Sephadex-C1 porphyrin (sample A) and CM Sephadex-taurine-C1 porphyrin (sample B) as cytocidal agents against microbial pathogens was comparatively investigated by using one typical representative of Gram-positive bacteria, namely meticillin-resistant Staphylococcus aureus (MRSA), and one typical representative of Gram-negative bacteria, namely Escherichia coli, as substrates. Toward this end, aqueous suspensions of both bacterial strains (10⁸ cells/ml) were exposed to full spectrum visible light in the presence of either sample A or sample B using a porphyrin concentration of 0.5 mg/ml. Before irradiation, the suspension was incubated at room temperature in the dark for 60 min. under gentle magnetic stirring. The survival of the bacterial cells at different light exposure times was determined by serial 10-fold dilution of an aliquot of the irradiated suspension with the growth medium, followed by plating in order to obtain the CFU/ml. The data on the decrease in the survival of the bacterial cells as a function of the irradiation time are summarized in Table 1.

**Table 1**

| *Decrease in survival (log scale) of a typical Gram-positive and Gram-negative bacterium ( 10⁸ cells*/*ml) upon irradiation (400-800 nm, 100 mW*/*cm²) in the presence of CM-Sephadex-C1 porphyrin (A) and CM-Sephadex-taurine-C1 porphyrin (B). The irradiations were performed after incubation of the two samples in neutral aqueous solution (PBS) for 60 min.* | | | | |
|---|---|---|---|---|
| **Irradiation time (min.)** | **Sample A** | | **Sample B** | |
| | **MRSA** | **E. *coli*** | **MRSA** | ***E. coli*** |
| 0 | 0.0 | 0.0 | 0.0 | 0.0 |
| 1 | -1.7 | -1.0 | -2.3 | -2.0 |
| 5 | -2.9 | -1.8 | -3.7 | -3.4 |
| 10 | -3.7 | -3.1 | -5.4 | -5.1 |
| 30 | -4.8 | -3.8 | -6.7 | -6.6 |

| | | | | |
|---|---|---|---|---|
| MRSA = Meticillin-resistant *Staphylococcus aureus* E. coli = *Escherichia coli* C1 concentration = 0.5 mg/ml | | | | |

Clearly, both samples, once photoexcited, can induce a several log decrease in the survival of the Gram-positive and Gram-negative bacterial strains. However, it is apparent that the extent of bacterial photoinactivation, for both MRSA and E. coli, is substantially larger in the case of the sample where the taurine moiety has been inserted between the Sephadex resin and the C1 porphyrin. The enhancement of the antibacterial activity was apparent already after exposure of the bacterial cells to visible light for 1 min and reached about two orders of magnitude, or even larger, at irradiation times of 30 min. This effect is in agreement with the higher fraction of porphyrin monomeric state, longer stability and greater yield of singlet oxygen generation which have been measured for the CM Sepadex-taurine-C1 porphyrin sample, underlining the appreciably positive influence of the spacer.

When the experiments on the photosensitised inactivation of MRSA and E. coli were repeated with samples A and B which had been kept in the aqueous suspension for 1 week before addition of the bacterial cells, a decrease in the extent of the cytocidal effect was clearly detectable for the CM Sephadex-C1 porphyrin adduct, while the data concerning the drop in survival for the Gram-positive and Gram-negative bacteria irradiated in the presence of the CM Sephadex-taurine-C1 porphyrin adduct were only marginally different from those obtained upon irradiation after only 60 min. incubation (Table 2).

**Table 2**

| *Decrease in survival (log scale) of a typical Gram-positive and Gram-negative bacterium (10⁸ cells*/*ml) upon irradiation (400-800 nm, 100 mW*/*cm² ) in the presence of CM-Sephadex-C1 porphyrin (A) and CM-Sephadex-taurine-C1 porphyrin (B). The irradiation was performed after incubation of the two samples in neutral aqueous solution (PBS) for one week.* | | | | |
|---|---|---|---|---|
| **Irradiation time (min.)** | **Sample A** | | **Sample B** | |
| | **MRSA** | ***E. coli*** | **MRSA** | *E. **coll*** |
| 0 | 0.0 | 0.0 | 0.0 | 0.0 |
| 1 | -1.1 | -0.7 | -2.2 | -2.0 |
| 5 | -2.0 | -1.2 | -3.4 | -3.5 |
| 10 | -2.9 | -2.6 | -5.4 | -5.0 |
| 30 | -3.5 | -3.2 | -6.6 | -6.3 |

| | | | | |
|---|---|---|---|---|
| MRSA = Meticillin-resistant *Staphylococcus aureus* E. coli *= Escherichia coli* C1 concentration = 0.5 mg/ml | | | | |

Thus, it is reasonable to conclude that the photoactivity of the taurine-containing adduct undergoes no significant decrease after one week.

### Example 3

### Photosensitised inactivation of bacteria under dynamic conditions

The studies about the ability of the CM Sephadex-taurine-C1 porphyrin adduct to disinfect water via the photosensitised killing of microbial contaminants was finally extended to a circulating flow photoreactor system, such as the one described in Figure 1. Toward this aim a 50 liter water sample, which had been artificially contaminated with 10⁶ MRSA cells/ml, was flowed upward through an irradiation chamber of cylindrical shape, equipped with a total number of 5 parallel nylon filters filled with the adduct. The upper and lower surface of each filter through which the water was passing was illuminated with a LED whose emission was peaking at 420 nm, using a light intensity of 25 mW/cm². The porphyrin concentration in the conjugate was 10 µM. The flow rate was changed between 5 liters/min and 30 liters/min. The overall transit time of water from the lowest to the top point of the irradiation chamber was 20 min. An efficient drop in the population of the bacteria in the flowing water was achieved, with a decrease in the cell colonies from 6.5 CFU/ml (unirradiated system) to 3.7 CFU/ml (irradiated, water flow rate of 5 liters/min) and 4.5 CFU/ml (irradiated, water flow rate 25 liters/min). No significant chemical modification (photobleaching) of the porphyrin was observed at the end of each irradiation session.

These data show that the C1 porphyrin immobilized on the resin through a spacer can efficiently induce the decrease in the survival of a highly pathogenic bacterium, such as MRSA, upon illumination with 420 nm light from a LED source of an infected water sample made to flow through the above described irradiation chamber.

## Claims

1. An adduct of general formula:
R-S-P
in which R is a resin carboxylated with a -(CH₂)ₙCOOH group wherein n is comprised between 1 and 3,
S is a spacer of general formula A - X - B,
wherein A is a moiety which covalently binds to said resin and it is selected from the group consisting of an amino group, an alcohol group and a thiol group,
X is (CH₂)_{z} or (O-CH₂-CH₂)_{y} wherein z is comprised between 1 and 20 and y is comprised between 1 and 50,
B is a negatively charged moiety which establishes a ionic link with the positively charged moieties of the component P of the adduct,
P is a porphyrinoid,
and wherein S is linked to R through a covalent bond and S is linked to P through an ionic bond.

2. The adduct according to claim 1, wherein said porphyrinoid is selected from the group consisting of porphyrin, chlorin, bacteriochlorin, porphycene, phthalocyanine and naphthalocyanine.

3. The adduct according to anyone of claims 1-2 in which said moiety B of said spacer S is selected from the group consisting of a carboxylate, a sulphonate and a phenolate group.

4. The adduct according to anyone of claims 1-3 in which said spacer S is taurine.

5. The adduct according to anyone of claims 1-4 in which P is a porphyrin of general formula (I) 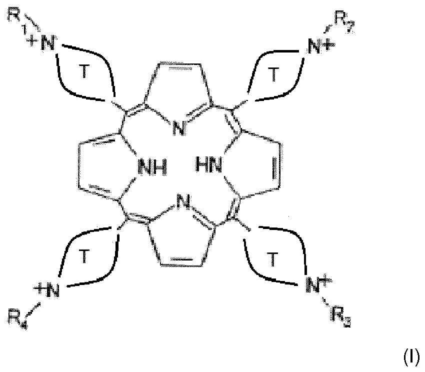 wherein
T is a heterocyclic ring selected from the group consisting of pyridyl, piperidyl and pyrimidyl; R₁=R₂=R₃ is -CH₃, and
R₄ is selected from the group consisting of straight or branched, saturated or unsaturated C₁-C₂₂ hydrocarbon chain; C₆-C₁₀ aromatic, C₆-C₁₀ heteroaromatic ring containing one or more heteroatoms selected from the group consisting of oxygen, sulphur and nitrogen, and/or mixed C₁-C₂₂ alkyl-C₆-C₁₀ aromatic moieties.

6. The adduct according to anyone of claims 1-5 wherein P is selected from the group consisting of meso-tetra(N,N,N,N-methyl-pyridyl)porphine (C1), meso-tri(N-methyl-pyridyl), mono(N-dodecyl-pyridyl)porphine (C12) and meso-tri(N-methyl-pyridyl), mono(N-tetradecyl-pyridyl)porphine (C14).

7. The adduct according to anyone of claims 1-6, wherein P further comprises a chelated metal ion selected from the group consisting of: Mg(II), Zn(II), Al(III), In(IV), Ge(IV), Si(IV), Sn(IV) and Pd(II).

8. A process for manufacturing the adduct of anyone of claims 1-7 comprising the steps of:
a) linking a spacer S to a resin R by adding an excess of said spacer in order to promote the formation of a covalent bond between said spacer and said resin;
b) adding an excess of a porphyrinoid P in order to form an ionic bond between the negatively charges of said spacer S and the positive charges of said porphyrinoid P;
c) removing the unbound porphyrinoid P by repeated washing.

9. An apparatus comprising an irradiation chamber (1) provided with an inlet channel (2') for the entrance of water, wherein means (6') for preventing contaminating materials entering and the beads of the resin-spacer-porphyrinoid adduct exiting is placed at the point of entrance of the inlet channel (2') in the irradiation chamber, and an outlet channel (2") for water exit, wherein means (6") for preventing passage of particulates is placed at the point of exit of the outlet channel (2") from the irradiation chamber, at least one light source (3) positioned inside said irradiation chamber (1), and at least one resin-spacer-porphyrinoid adduct of anyone of claims 1-7 within said irradiation chamber (1).

10. The apparatus according to claim 9 wherein said at least one light source (3) is fixed on the inside walls of said irradiation chamber (1) or is included in transparent tubes (5), said tubes being fixed on the inside walls of said irradiation chamber (1).

11. The apparatus according to anyone of claims 9-10, wherein said irradiation chamber (1) further comprises one or more filters (4) and said resin-spacer-porphyrinoid adduct is included in said one or more filters (4).

12. The apparatus according to anyone of claims 9-10 wherein said at least one resin-spacer-porphyrinoid adduct is loaded by affinity binding on a tissue.

13. The apparatus according to anyone of claims 9-10, wherein said at least one resin-spacer-porphyrinoid adduct is dispersed inside said irradiation chamber (1), said apparatus further comprising a compressed air supplier (9) connected to said irradiation chamber (1) through channels (8) for the entrance of air, an air outlet device (12) for air exit, thus allowing the formation of an influx of air (11) counteracting the water flow, one or more mechanical stirrers (7) and one or more deflectors (10) disposed on opposite inside walls of said irradiation chamber (1).

14. A system comprising the apparatus of anyone of claims 9-13 in which water is disinfected, a collector of the disinfected water, a hydraulic piping connecting the elements, a control panel and optionally one or more of the following elements: a buffering tank in which water is collected before flowing into said apparatus, a manometer, a flowmeter, hydraulic valves, a centrifugal pump and a frequency inverter.

15. Use of the apparatus of anyone of claims 9-13 or of the system of claim 14 for disinfecting water from microbial species.
